(19) Europäisches Patentamt
European Patent Office
Office européen des brevets

(11) Numéro de publication: **0 094 330**
**A1**

(12) **DEMANDE DE BREVET EUROPEEN**

(21) Numéro de dépôt: **83440026.9**

(22) Date de dépôt: **29.04.83**

(51) Int. Cl.³: **A 43 C 9/04**

(30) Priorité: **10.05.82 FR 8208527**

(43) Date de publication de la demande: **16.11.83**
**Bulletin 83/46**

(84) Etats contractants désignés: **AT BE CH DE GB IT LI LU NL SE**

(71) Demandeur: **SARL TREFICOM, 129 rue de Dunkerque, F-59200 Tourcoing (Nord) (FR)**

(72) Inventeur: **Crombez, Charles Léon, 951 Bois d'Achelles, F-59223 Roncq (Nord) (FR)**

(74) Mandataire: **Lepage, Jean-Pierre, c/o BUGNION PROPRIETE INDUSTRIELLE SARL 23/25, rue Nicolas Leblanc, F-59011 Lille Cédex 1 (Nord) (FR)**

(54) **Une cordelette, son procédé de traitement ainsi qu'une installation de mise en oeuvre du procédé.**

(57) L'invention est relative à une cordelette, un procédé de traitement de la cordelette, une installation de mise en oeuvre du procédé de traitement. La cordelette, selon l'invention, présentera au moins une partie dans laquelle les éléments unitaires la composant sont resserrés et adhèrent mutuellement entre eux. Les éléments unitaires des cordelettes (8) sont resserrés par traction de la cordelette entre les deux paires de rouleaux (11) et (12) et (9) et (10) puis soudés au moins superficiellement par air chaud et enfin coupés.

L'invention trouvera notamment son application dans la fabrication industrielle d'embouts de lacets, de cordons de sangles.

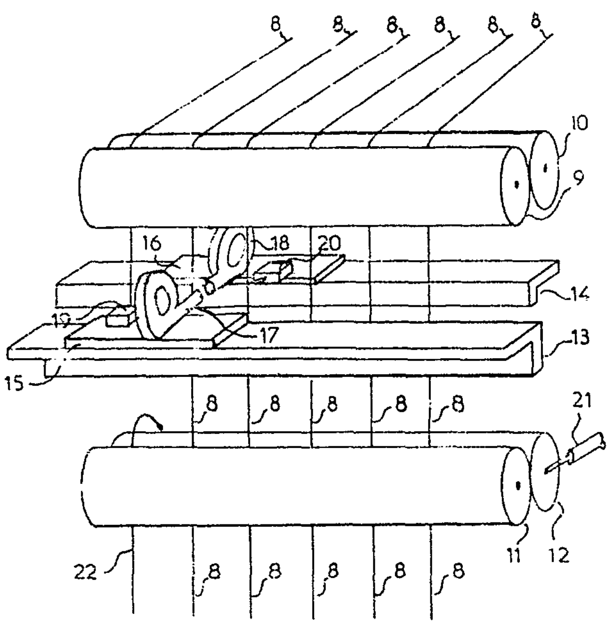


EP 0 094 330 A1

L'invention est relative à une cordelette, un procédé de traitement de la cordelette et une installation pour la mise en oeuvre du procédé. Elle trouvera notamment son application parmi les lacets, cordons et sangles, ainsi que la formation de leurs embouts.

Les cordelettes sont généralement composées de fils tressés, cablés ou retors. Ces fils peuvent être composés de fibres naturelles, synthétiques ou mélangées.

Actuellement, pour éviter l'effilochage de leurs extrémités, les cordelettes disposent à leurs extrémités de moyens connus, tels qu'un noeud, une agrafe métallique ou un tube de cellophane collé.

Le noeud présente l'inconvénient de provoquer une surépaisseur locale qui limite son application et lui interdit par exemple d'être utilisé dans les lacets. L'agrafe métallique, utilisée comme embout de lacet, présente des risques d'oxydation, et demande à être peinte de la même couleur que le lacet pour des raisons d'esthétique. La garniture de cellophane, enveloppant l'extrémité des lacets est fragile, car elle présente des risques de déchirement ou de décollage. Sur le plan fabrication, les embouts métalliques ou de cellophane présentent également de nombreux inconvénients. La pose de ces embouts est unitaire et ils doivent être adaptés de façon précise au diamètre des lacets.

Pour les sangles constituées de fils textiles et synthétiques, la coupe peut avantageusement être réalisée à l'aide d'une lame chaude, ainsi sur la section tranchée, les fils sont fondus et soudés ensembles. Toutefois, l'application de ce procédé reste limitée, étant donnés les défauts qu'il engendre. La fusion qui n'est pas contrôlée, provoque une déformation et une surépaisseur, visible et généralement d'un bourrelet, dans la périphérie de la section coupée. Pour faciliter la découpe, la lame est généralement chauffée à haute température et provoque un noircissement de la tranche, préjudiciable à l'esthétique du produit. De plus, seule la section tranchée est rigidifiée par soudage des fils et donc la partie rigide est insuffisante pour être utilisée comme embout de lacet.

Le but principal de la présente invention est de proposer une cordelette qui présente au moins une partie traitée qui peut servir, grâce à sa rigidité, d'embout de lacet présentant toutes les facilités pour l'introduire dans les oeillets de chaussures, et qui évite l'effilochage de l'extrémité. La partie traitée conserve la teinte de la cordelette d'origine et présente une bonne solidité, ce qui la prémunit

de tout déchirement accidentel, alliant ainsi les qualités des embouts de lacets connus actuellement, sans en présenter les inconvénients.

Un autre but de la présente invention est de proposer un procédé de traitement de la cordelette.

Le procédé selon l'invention permet d'immobiliser les fils constituant la cordelette de façon groupée, ce qui évite l'effilochage des fils, et leur donne une certaine rigidité, ce qui permet d'enfiler facilement le lacet dans les oeillets de chaussures, lorsque la partie traitée est utilisée comme embout de lacet.

Un autre but de la présente invention est de proposer une installation capable de traiter plusieurs portions de cordelettes, mises en parallèle, selon le procédé de l'invention. L'installation est particulièrement adaptée, grâce à sa grande capacité de production, aux fabrications en série à faible coût de revient.

D'autres buts et avantages de la présente invention apparaîtront au cours de la description qui va suivra, qui n'est donnée cependant qu'à titre indicatif, et qui n'a pas pour but de limiter l'invention.

La cordelette composée de fils de fibres textiles naturelles, synthétiques ou mélangées notamment tressés, cablés ou retors en particulier destinés à la confection des lacets, sangles ou cordons, les fils unitaires distincts, groupés et ordonnés pouvant présenter entre eux des interstices, est caractérisée par le fait qu'elle présente au moins une partie dans laquelle les fils sont resserrés et des moyens pour les maintenir dans cette position en les faisant adhérer les uns aux autres.

L'invention sera mieux comprise si l'on se réfère à la description ci-dessous, ainsi qu'aux dessins en annexe qui en font partie intégrante.

La figure 1 schématise une coupe longitudinale de la cordelett selon l'invention.

La figure 2 schématise la coupe de la cordelette destinée à former des lacets.

La figure 3 schématise la coupe de la cordelette présentant des embouts fantaisie.

La figure 4 présente un autre type de cordelette munie d'embou fantaisie.

La figure 5 schématise une installation de traitement des cor-

delettes, selon le procédé de l'invention, particulièrement adaptée à la fabrication industrielle de séries.

La figure 5 illustre une seconde installation de traitement de la cordelette selon le procédé de l'invention.

La cordelette sera composée de fils unitaires qui pourront être tressés, cablés ou retors selon les procédés actuellement connus. Les fils seront généralement réalisés à partir de fibres textiles naturelles, synthétiques ou mélangées, mais l'invention pourra s'étendre également à toutes les compositions d'éléments unitaires distincts qui sont groupés et qui présentent entre eux des interstices tels que par exemple les cables, filins, tresses.

Selon l'invention, la cordelette, obtenue à partir d'un assemblage de fils unitaires illustrés à la figure 1 sous le repère 1, présentera une partie 2 dans laquelle les fils unitaires sont resserrés. Dans cette partie 2, les fils perdent leur individualité pour adhérer les uns aux autres.

Dans le cas de la figure 1, les fils de la partie traitée 2 ne présentent une allure compacte qu'en surface, l'âme 3 de la cordelette dans la partie considérée, est formée de fils comprimés par la gaine extérieure 4 formée des fils immobilisés en position resserrée par adhérence mutuelle.

Il est préférable que les fils dans la partie traitée soient resserrés, car dans cette position, ils sont en contact plus étroit avec leurs voisins, ce qui améliore l'adhérence mutuelle en présentant de plus grandes surfaces de contact. La compacité des fils dans la gaine 4 qui empêche tout déplacement individuel des fils donne une certaine rigidité à la partie traitée. Cette rigidité est d'autant moins grande que la partie de la cordelette dans laquelle les fils adhèrent les uns aux autres, est superficielle. Le resserrage des fils peut provoquer, comme dans le cas de la figure 1, un rétrécissement de la section de la cordelette 1 dans la partie traitée ; ce rétrécissement peut être nul si la cordelette présente dès l'origine des fils serrés ne présentant pratiquement aucun interstice entre eux.

Si la zone traitée 2 se trouve à l'extrémité de la cordelette, ce qui peut être obtenu en sectionnant la cordelette au niveau de la zone traitée 2 comme dans le cas de la figure 2, la gaine 4 des fils adhérant mutuellement au moins en surface de la cordelette, évite tout effilochage de l'extrémité, la partie traitée 2 peut servir par

BAD ORIGINAL

exemple d'embout de lacet, elle possède en effet les qualités de rigidité nécessaires pour enfiler facilement le lacet dans les oeillets de chaussures.

Dans le cas des sangles, qui en raison généralement de leur faible épaisseur, peuvent présenter une partie traitée qui atteindra l'âme de la sangle, ces parties traitées de préférence localisées aux extrémités de la sangle interdiront tout effilochage et faciliteront avantageusement le passage de la sangle dans le coulants.

La figure 3 présente le cas d'une cordelette 1 qui présente deux parties traitées, 5 et 6 voisines et qui a été coupée entre ces deux parties. Les extrémités coupées s'effilochent jusqu'aux parties traitées et forment des franges. Il est ainsi possible de créer des embouts fantaisie.

A la figure 4, la cordelette présente des parties déformées selon un procédé connu comme l'introduction d'une bille à l'intérieur de la cordelette lors du tressage de telle sorte qu'elle forme une excroissance 7 qui pourra être coupée de façon à former des embouts, après traitement selon l'invention.

Ainsi, il est possible de réaliser des cordons ayant les mêmes propriétés que ceux présentant des noeuds à leurs extrémités et qui sont particulièrement appréciables dans le cas de cordons passés dans les ourlets de vêtements afin qu'aucune des extrémités du cordon ne rentre dans l'ourlet.

L'adhérence mutuelle des fils peut avantageusement être réalisée par soudage des fils dans le cas notamment de fils textiles synthétiques. En effet, lorsque les fils textiles, tels que ceux en polyester, sont portés à une température sensiblement voisine de leur température de fusion, ils fondent en prenant une allure pâteuse, cette fusion permet de souder les fils après refroidissement.

Dans le cas des fibres naturelles, l'adhérence pourra par exemple être obtenue par une imprégnation préalable de la cordelette au niveau des parties à traiter, d'un vernis qui après séchage ou polymérisation de préférence par chauffage, maintiendra les fils immobilisés de façon homogène.

Le traitement d'une partie de la cordelette pourra être réalisé selon le procédé non limitatif suivant. On resserre les fils, au moins dans la partie à traiter, composant la cordelette par un procédé classique qui peut être par exemple un pincement entre deux matrices complémentaires, mais ce choix oblige à disposer d'un jeu de matrices pour chaque format de cordelette, aussi est-il préférable d'assurer le resserrage des fils, au moins dans la zone à traiter, par une traction exercée sur la cordelette de part et d'autre de la partie à traiter. La tension applique les fils enchevetrés les uns sur les autres et les resserre en comblant au moins partiellement les interstices existants entre les fils unitaires. La tension présente également l'avantage de laisser la partie à traiter dégagée.

[illegible] qu'on provoque un échauffement [illegible] jusqu'à [illegible] pour que les fils se soudent [illegible] il est en général inutile de souder les fils qui composent l'âme de la cordelette dans la partie à traiter, une fusion et donc un soudage superficiel est suffisant pour éviter l'effilochage et donner une certaine rigidité à la zone traitée.

L'échauffement, selon l'invention, pourra être réalisé par tout procédé connu et en particulier avantageusement par soufflage de gaz chaud sur la partie à traiter. Pour ces raisons de commodité, le gaz pourra être de préférence de l'air. L'air chaud pourra être éjecté à partir d'une buse de forme appropriée sur la partie de la cordelette à traiter. Deux ou plusieurs buses pourront avantageusement être utilisées et réparties autour de la cordelette dans la zone à traiter afin de provoquer un échauffement plus homogène de la périphérie de la cordelette dans la zone à traiter par une meilleure répartition des surfaces d'impact de l'air chaud sur la cordelette.

La chaleur apportée à la surface de la cordelette par le contact de l'air, pénêtrera vers l'âme de la cordelette. Le réglage de la température et du temps d'application de l'air chaud sur la cordelette seront ajustés de telle sorte qu'il y ait fusion et soudure des fils au moins en surface sans qu'il y ait jaunissement ou noircissement de la cordelette dû à une température trop élevée ou un temps d'application trop prolongé.

Pour des impératifs industriels de production, il est préférable de réduire au maximum le temps d'application de l'air chaud sur les cordelettes, en contrepartie, il est nécessaire d'utiliser de l'air très chaud. De bons résultats ont été obtenus dans la fabrication d'embouts de lacets en polyester avec de l'air soufflé à 600 degrés pendant environ une seconde.

L'utilisation d'un air très chaud avec un temps d'application court limite la soudure des fils composant la cordelette à ceux situés

BAD ORIGINAL

en surface de la zone traitée, la chaleur n'ayant pas le temps de se transmettre à l'intérieur de la cordelette. Il se forme alors une gaine superficielle, formée par les fils soudés mutuellement, alors que le centre de la cordelette dans la partie traitée est formé de fils unitaires comprimés, analogues à la gaine 4 et à l'âme 3 de la figure 1.

Dans le cas des textiles naturels, une enduction préalable de vernis est nécessaire. Pour cela, on dépose par un procédé connu du vernis sur la partie à traiter par exemple à l'aide d'un rouleau caoutchouté imbibé de vernis ; la cordelette s'imprègne du vernis, puis on resserre les fils par traction comme décrit précédemment et enfin on polymérise ou on sèche le vernis qui maintiendra ainsi les fils regroupés et donnera une certaine rigidité à la partie traitée.

La polymérisation ou le séchage du vernis sera avantageusement provoqué ou accéléré par échauffement de la partie à traiter. L'échauffement pourra être réalisé, de préférence, par soufflage de gaz chaud selon le procédé décrit précédemment.

Pour les textiles mélangés, suivant la composition, le vernis ne sera éventuellement pas nécessaire et l'on pourra utiliser le procédé de traitement analogue aux textiles synthétiques.

La cordelette pourra ensuite, selon le procédé, être coupée selon les besoins. La coupe pourra être réalisée par tout procédé connu comme par exemple une lame ou des ciseaux motorisés, ou par un jet d'air chaud localisé qui provoquera une fusion dans sensiblement toute la section de la cordelette.

Une installation adaptée à la fabrication industrielle d'embout de lacet ou de cordon selon le procédé décrit est schématisée à la figure 5.

Des cordelettes 8 provenant par exemple de bobines installées sur un dévidoir, sont pincées en parallèle entre deux rouleaux 9 et 10, par gravitation, les extrémités des cordelettes 8 descendent entre deux autres rouleaux 11 et 12, placés à la verticale des premiers, susceptibles de se rapprocher pour pincer également les cordelettes 8.

Entre les deux paires de rouleaux 9 et 10, 11 et 12, de part et d'autre se trouvent des bancs, dans le cas de la figure deux bancs 13 et 14, sensiblement horizontaux, sur lesquels coulissent des chariots 15 et 16. Ces chariots pouvant être entraînés par tout dispositif approprié, en particulier une vis mère motorisée. Des dispositifs de soufflage de gaz chaud, dans le cas de la figure des pistolets à air chaud

17 et 18, ainsi des ciseaux motorisés 19 et 20 sont fixés sur chacun des chariots 15 et 16.

L'installation décrite, présente deux bancs 13 et 14 car ils donnent de bons résultats mais pour certaines applications, un ou plusieurs bancs peuvent être également envisagés.

Dans un premier temps, les cordelettes 8 pincées dans les rouleaux 9 et 10 sont tirées du dévidoir par ces deux rouleaux 9 et 10 entraînés de façon appropriée par un moteur asservi par exemple ; les cordelettes 8 descendent par gravitation entre les deux rouleaux inférieurs 11 et 12 qui sont dans cette phase du cycle écartés l'un de l'autre, par un dispositif approprié tel qu'un vérin 21.

Lorsque la longueur de la cordelette descendue correspond à celle du lacet à réaliser, les rouleaux supérieurs 9 et 10 s'arrêtent et se bloquent dans cette position.

Alors, les rouleaux inférieurs 11 et 12 se rapprochent mutuellement et pincent la cordelette. Le rapprochement étant réalisé par un dispositif approprié tel que le vérin 21.

Les rouleaux inférieurs 11 et 12 et supérieurs 9 et 10 présenteront, de préférence, un bon coefficient de frottement vis-à-vis de la cordelette et pourront par exemple être caoutchoutés.

Ensuite, un dispositif approprié exercera un couple sur au moins un des rouleaux inférieurs, par exemple par l'intermédiaire d'un moteur couple, de façon à venir exercer une traction sur les cordelettes retenues par les rouleaux supérieurs 9 et 10.

Cette mise en tension des cordelettes, permet de resserrer les fils de la cordelette situés entre les deux paires de rouleaux inférieurs et supérieurs.

Puis, les chariots 15 et 16 se déplacent sur toute la longueur des bancs 13 et 14. Les pistolets à air chaud 17 et 18 viennent chauffer et souder par une fusion au moins superficielle les fils successivement de toutes les cordelettes, selon le procédé de traitement précédemment décrit.

Les buses des pistolets à air chaud seront adaptées suivant la longueur de la partie traitée que l'on désire. La largeur horizontale des buses et la vitesse de déplacement des chariots permettent d'ajuster le temps d'application de l'air chaud sur chaque cordelette. Par exemple pour les embouts de lacets : les buses seront, de préférence, ovalisées, de largeur horizontale sensiblement constante, le grand axe étant ver-

BAD ORIGINAL

tical.

Puis les ciseaux motorisés 19 coupent les parties traitées, sensiblement dans leur milieu pour former des embouts de lacets.

Lorsque les chariots 15 et 16 arrivent en fin de course à l'extrémité des bancs 13 et 14, toutes les cordelettes ont été coupées et forment des lacets.

Les ciseaux seront de préférence placés à une distance des pistolets à air chaud telle que les parties traitées soient coupées alors qu'elles sont refroidies, pour que la coupe soit nette.

Ensuite, les rouleaux inférieurs 11 et 12 s'écartent par l'intermédiaire par exemple du vérin 21 ; les lacets, tels que celui représenté à la figure 5 sous le repère 22 tombent et sont recueillis par un dispositif de conditionnement approprié.

Un autre cycle de fabrication analogue à celui décrit peut recommencer, mais cette fois ci les chariots 15 et 16 parcoureront les bancs 13 et 14 dans le sens opposé et ce seront les ciseaux mécanisés 20 qui couperont les parties traitées.

Le système de coupe aurait pu être réalisé au moyen d'un second pistolet à air chaud ou d'une forme de buse telle qu'elle localise plus longtemps sur une portion de la cordelette, le jet d'air chaud afin de provoquer la fusion complète de la section ce qui aurait entraîné la coupure de la cordelette.

Pour les textiles naturels, les chariots auraient pu disposer d'un système par exemple un rouleau imbibé de vernis qui aurait été déposé sur les cordelettes avant le passage des pistolets à air chaud.

L'installation décrite est particulièrement adaptée aux fabrications en série bien qu'il ne soit pas nécessaire que toutes les cordelettes soient du même type.

La température de l'air chaud, la vitesse de défilement des chariots ainsi que les dimensions des buses, les pistolets à air chaud seront adaptés selon la matière et les dimensions des cordelettes à traiter.

Une autre installation de mise en oeuvre du procédé selon l'invention est proposée, à titre non limitatif, à la figure 6.

L'installation est conçue pour réembobiner la cordelette qui a subi le traitement selon le procédé, afin que celle-ci soit coupée ultérieurement selon les besoins de l'utilisateur. Un rembobinage de la cordelette aurait pu également être envisagé sur l'installation pré-

BAD ORIGINAL

cédente, en supprimant la coupe et en rembobinant les cordelettes à la sortie de leur passage dans les rouleaux inférieurs 11 et 12.

L'installation schématisée à la figure 6, représente une bobine, dans le cas de la figure, de forme sensiblement parallélépipédique. Des poulies 23, 24, 25, 26, 27, 28, 29 et 30 sont placées aux coins du parallélépipède, légèrement à l'intérieur de celui -ci, des courroies 31, 32, 33, 34 sont placées entre les poulies de telle sorte qu'elles forment les arêtes parallèles du parallélépipède, le retour des courroies se faisant à l'intérieur du parallélépipède.

Une broche 35 enroule la cordelette, provenant d'un dévidoir, autour du parallélépipède, les spires formées reposant sensiblement perpendiculaires sur les courroies qui forment les arêtes parallèles du parallélépipède.

Les courroies 31, 32, 33 et 34 sont entraînées dans la même direction, à la même vitesse par tout moyen approprié tel qu'un moteur entraînant les poulies.

Ainsi, les spires progressent sur des arêtes du parallélépipède puis à l'aide d'une seconde broche 36, les spires sont déroulées du parallélépipède et rembobinées selon des moyens connus.

Dans un mode préférentiel de réalisation, les courroies seront légèrement divergentes afin de mettre sous tension progressivement les spires au cours de leur déplacement sur les arêtes du parallélépipède formées par les courroies en déplacement.

Un ou plusieurs pistolets à air chaud 37 seront fixés de telle sorte que leur buse dirige leur jet d'air sur une partie des spires sous tension de façon à produire une fusion et ainsi une soudure au moins superficielle des fils de la cordelette dans la partie 33 traitée selon le procédé précédemment décrit.

Un système de coupage de la spire, après qu'elle ait été trai aurait pû être envisagé pour obtenir des lacets ou cordons analogues à la première installation.

De même un dispositif d'imprégnation de vernis de la cordelette, en particulier pour celle réalisée à partir de textiles naturels, aurait pû être réalisé en disposant par exemple d'un rouleau imbibé de vernis en contact avec les spires de la cordelette en amont du pistolet à air chaud.

Le réglage de la dimension des spires par modification de l'écartement des poulies permet d'adapter le dispositif selon l'interv

BAD ORIGINAL

entre deux parties traitées, désirées.

La vitesse de défilement des courroies, la dimension des buses et la température de l'air chaud seront ajustées selon le type de cordelettes à traiter.

Le mode de réalisation qui vient d'être décrit n'est donné qu'à titre indicatif, et d'autres mises en oeuvre de la présente invention, à la portée de l'Homme de l'Art, auraient pû être adoptées sans pour autant sortir du cadre de celle-ci.

REVENDICATIONS

1. Cordelette composée de fils de fibres textiles naturelles, synthétiques ou mélangées notamment tressés, cablés ou retors en particulier destinés à la confection de lacets, sangles ou cordons, les fils unitaires distincts, groupés pouvant présenter entre eux des interstices, caractérisée par le fait qu'elle présente au moins une partie dans laquelle les fils sont resserrés et des moyens pour les maintenir dans cette position en les faisant adhérer les uns aux autres.

2. Cordelette composée de fils unitaires selon la revendication 1, caractérisée par le fait que les moyens permettent de rigidifier la cordelette dans la partie de la cordelette où les fils sont resserrés et adhèrent les uns aux autres dans cette position.

3. Cordelette composée de fils unitaires selon la revendication 1, caractérisée par le fait que l'adhérence entre les fils, est localisée sur une couche périphérique qui forme une gaine (4) sur une partie de la cordelette (1).

4. Cordelette composée de fils unitaires selon la revendication 1, caractérisée par le fait qu'elle présente une zone rétrécie dans laquelle les fils unitaires adhèrent les uns aux autres.

5. Cordelette composée de fils unitaires selon la revendication 1, caractérisée par le fait qu'elle présente des franges entre son extrémité et la partie traitée.

6. Procédé de traitement d'une partie de cordelette selon la revendication 1, constitué d'éléments unitaires notamment tressés cablés ou retors, destinés par exemple à l'obtention d'embouts de lacets, caractérisé en ce que l'on resserre des éléments unitaires dans la zone de traitement puis que l'on immobilise les fils dans cette position rapprochée et que l'on réalise une adhérence au moins partielle des éléments unitaires entre eux.

7. Procédé de traitement selon la revendication 6, caractérisé par le fait que l'on resserre les éléments unitaires par une traction exercée sur la cordelette de part et d'autre de la zone de traitement.

8. Procédé de traitement selon la revendication 6, caractérisé par le fait que l'on réalise une adhérence mutuelle des éléments unitaires superficiels de la cordelette.

9. Procédé de traitement selon la revendication 6, caractérisé par le fait que l'adhérence est réalisée au moyen d'une soudure par fusion des éléments unitaires de la cordelette au moyen d'un jet de gaz

BAD ORIGINAL

chaud soufflé localement sur la partie à traiter.

10. Procédé de traitement selon la revendication 6, caractérisé par le fait que l'on coupe, en tronçons la cordelette au niveau de la partie traitée, sensiblement en son milieu, pour former des embouts.

11. Procédé de traitement [illegible] revendication [illegible] par le fait que l'on déforme [illegible] la cordelette [illegible] telle que l'introduction [illegible] de la cordelette lors de [illegible] laquelle les fils [illegible] par une traction [illegible] que [illegible] à un niveau quelconque [illegible] déformée pour qu'elle forme un embout.

12. Procédé de traitement selon la revendication 6, caractérisé par le fait que l'on dépose préalablement un vernis sur les zones à traiter pour enduire les fibres qui seront immobilisées par ce vernis lors de son séchage ou polymérisation.

13. Procédé de traitement selon les revendications 9 et 10, caractérisé par le fait que l'on coupe la cordelette au moyen d'un jet de gaz chaud qui provoque une fusion locale dans sensiblement toute la section de la cordelette.

14. Installation pour la mise en oeuvre du procédé selon la revendication 6, caractérisée par le fait qu'elle présente des moyens pour resserrer les éléments unitaires composant la cordelette dans la partie à traiter et des moyens pour provoquer une adhérence mutuelle des éléments unitaires de la partie à traiter.

15. Installation pour la mise en oeuvre du procédé selon la revendication 14, caractérisée par le fait que les moyens pour resserrer les éléments unitaires se présentent sous la forme de deux cylindres (9) et (10) entre lesquels sont pressées les cordelettes, et de deux autres cylindres (11) et (12), de préférence situés à la verticale des précédents, entre lesquels sont pressées les cordelettes, actionnés par un asservissement approprié de façon à mettre les cordelettes en traction avec les cylindres précédents (9) et (10).

16. Installation pour la mise en oeuvre du procédé selon la revendication 15, caractérisé par le fait que les cylindres (11) et (12) peuvent s'écarter l'un de l'autre pour faciliter l'introduction et le dégagement des cordelettes [illegible]

17. Installation pour la mise en oeuvre du procédé selon [illegible]

BAD ORIGINAL

revendication 14, caractérisée par le fait que les moyens provoquant l'adhérence des éléments unitaires se présentent sous la forme d'un dispositif balayant de l'air chaud, successivement sur les parties des cordelettes à traiter, de façon à provoquer une soudure au moins superficielle des éléments unitaires.

18. Installation pour la mise en oeuvre du procédé selon la revendication 17, caractérisée par le fait que le dispositif se présente sous la forme d'un ou plusieurs bancs (13) et (14) sur lesquels circulent des chariots (15) et (16) supportant des pistolets à air chaud (17) et (18).

19. Installation pour la mise en oeuvre du procédé selon la revendication 18, caractérisée par le fait qu'un dispositif approprié coupe les cordelettes après qu'elles aient subi le passage du ou des pistolets à air chaud.

20. Installation pour la mise en oeuvre du procédé selon la revendication 18, caractérisé par le fait que les pistolets à air chaud présentent une buse de forme appropriée pour provoquer une adhérence au moins superficielle de la partie de la cordelette à traiter et telle qu'elle provoque une fusion locale sensiblement sur toute la section de la cordelette pour la couper.

21. Installation pour la mise en oeuvre du procédé selon la revendication 14, caractérisée par le fait queles moyens pour resserrer les éléments unitaires se présentent sous la forme d'un ensemble de courroies (31, 32, 33, 34) disposées relativement parallèlement entre elles selon les arêtes d'un parallélogramme avec un léger angle de divergence, entraînées dans la même direction par tout dispositif approprié par exemple par des poulies situées au niveau des angles du parallélogramme, une broche (35) enroulant, autour de cet ensemble de courroie formant un parallélogramme, la cordelette sous forme de spires, ces dernières étant sensiblement perpendiculaires aux courroies.

22. Installation pour la mise en oeuvre du procédé selon la revendication 21 , caractérisée par le fait que les moyens pour provoquer l'adhérence des éléments unitaires composant la cordelette se présentent sous la forme d'un ou plusieurs jets de gaz chaud émettant leur souffle en direction d'une partie des spires de cordelettes enroulées autour du parallélogramme.

1/2

0094330

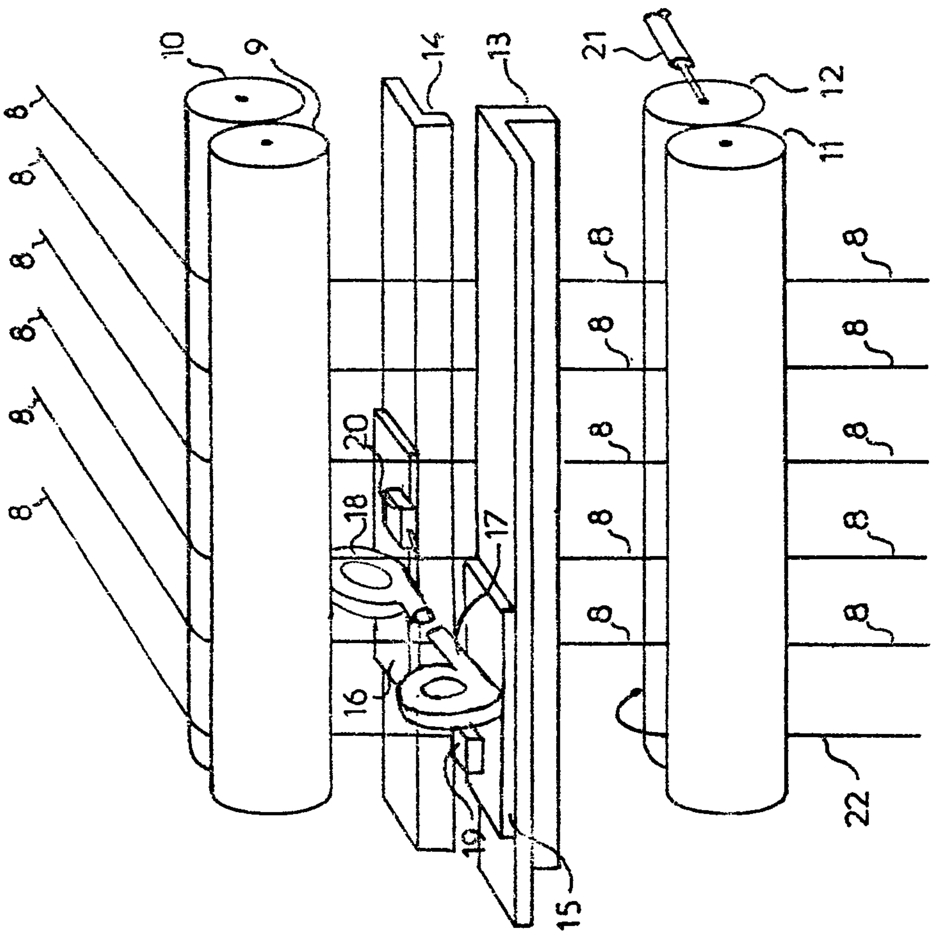


Fig 5

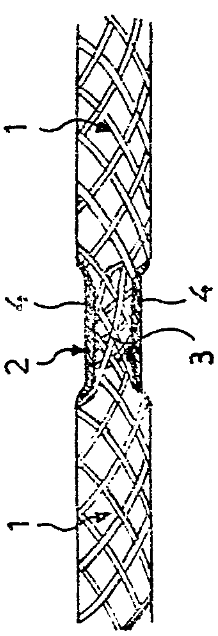


Fig 1

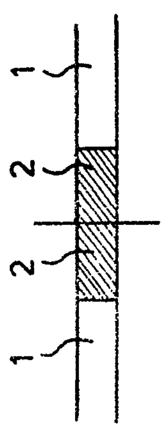


Fig 2

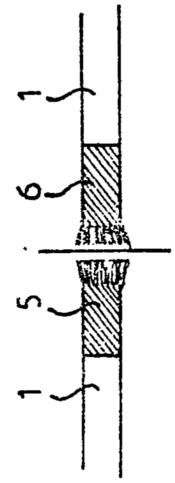


Fig 3

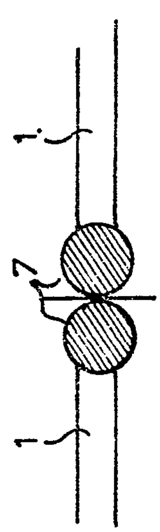


Fig 4

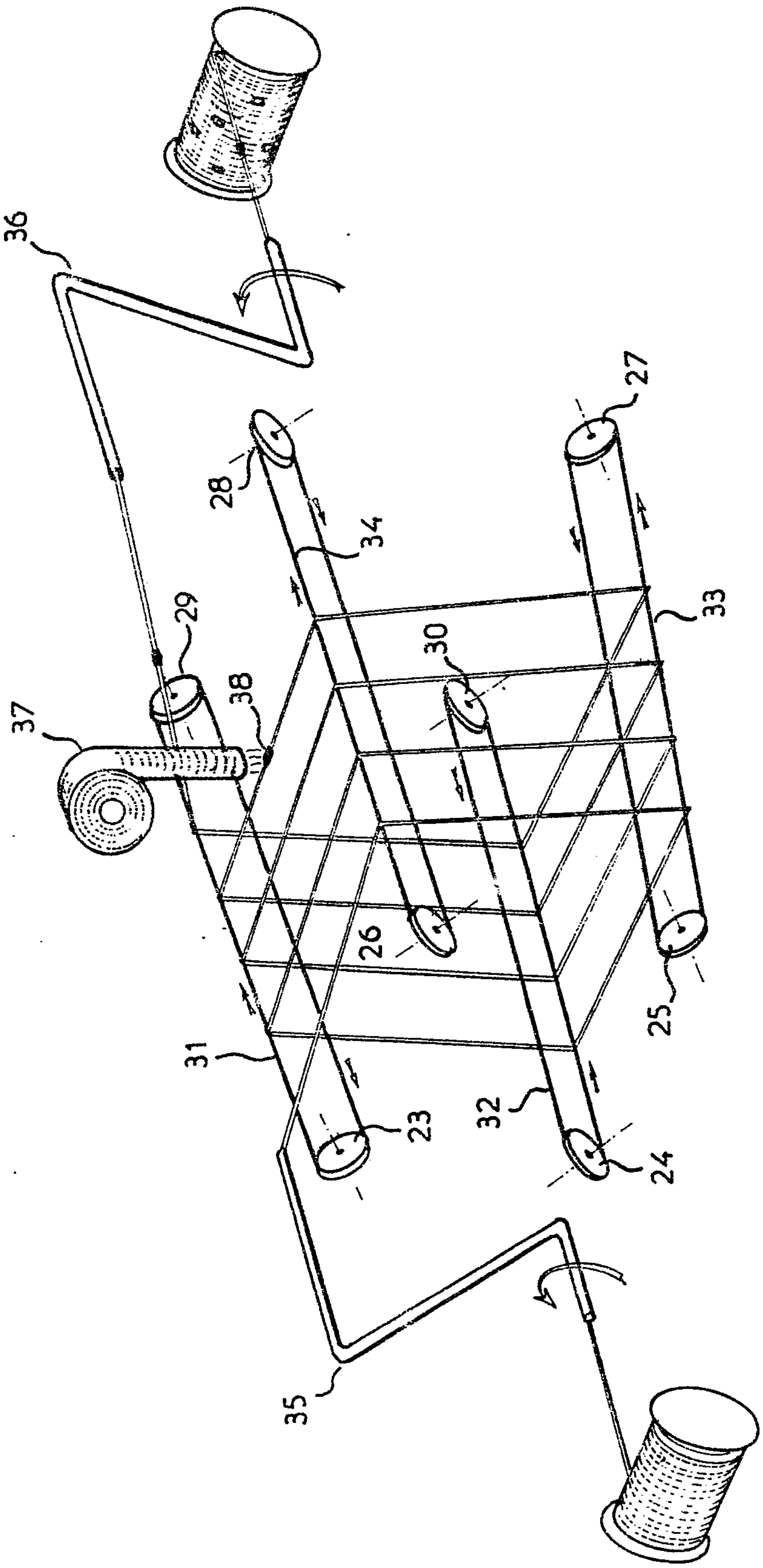


Fig 6

0094330

Office européen des brevets

# RAPPORT DE RECHERCHE EUROPEENNE

Numero de la demande

EP 83 44 0026

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | FR-A-2 367 591 (THOMAS TAYLOR)<br>* Revendications 1,2; page 4, lignes 6-20; figures 1-3 *<br>--- | 1,2,4, 6,10, 14 | A 43 C 9/04 |
| X | GB-A- 20 627 (WARDWELL) (A.D. 1911)<br>* Revendications 1,2,6; page 2, lignes 1-35; figures 1-4 *<br>--- | 1,6,11 ,12 | |
| A | US-A-3 323 165 (MOTTERN)<br>* Revendication 1; colonne 4, ligne 66 - colonne 6, ligne 21; figure unique *<br>--- | 1,7,15 ,16 | |
| | | | DOMAINES TECHNIQUES RECHERCHES (Int. Cl. 3) |
| A | FR-A- 909 448 (FRIOSSANT)<br>--- | | A 43 C<br>D 07 B<br>D 04 C<br>D 01 D |
| A | DE-C- 137 290 (ALBRECHT)<br>--- | | |
| A | DE-C- 466 642 (OVERBECK)<br>----- | | |

Le présent rapport de recherche a été établi pour toutes les revendications

| Lieu de la recherche | Date d'achèvement de la recherche | Examinateur |
|---|---|---|
| LA HAYE | 05-08-1983 | VAN GELDER P.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

BAD ORIGINAL